Europäisches Patentamt

(19) European Patent Office

Office européen des brevets

(11) Numéro de publication: **0 100 734**
**B1**

(12) ## FASCICULE DE BREVET EUROPÉEN

(45) Date de publication du fascicule du brevet:
17.12.86

(51) Int. Cl.⁴: **A 61 K 35/22,** A 61 K 37/02

(21) Numéro de dépôt: 83401578.6

(22) Date de dépôt: 29.07.83

(54) **Procédé de séparation ou de purification d'un facteur favorisant le sommeil à partir d'un milieu biologique par une réaction immunologique du type antigène-anticorps.**

(30) Priorité: 30.07.82 FR 8213410

(43) Date de publication de la demande:
15.02.84 Bulletin 84/7

(45) Mention de la délivrance du brevet:
17.12.86 Bulletin 86/51

(84) Etats contractants désignés:
AT BE CH DE GB IT LI LU NL SE

(56) Documents cité:
US-A-4 342 748

BIOLOGICAL ABSTRACTS, vol. 70, 1980, abrégé no. 5706, Philadelphia, Pa., USA,J.M. KRUEGER et al.: "Sleep-promoting material from human urine and its relation to factor S from brain"
FEDERATION PROCEEDINGS, vol. 38, no. 3, partie 1, 1979, page 1130, abrégé no. 4771,J. KRUEGER et al.: "Extraction of sleep-promoting material (SPU) from human urine"

(73) Titulaire: **ANVAR Agence Nationale de Valorisation de la Recherche, 43, rue Caumartin, F-75436 Paris Cédex 09 (FR)**

(72) Inventeur: **Bahr, Georges, 84, Boulevard Pasteur, F-75015 Paris (FR)**
Inventeur: **Chedid, Louis, 16- 18, rue Gaston de Caillavet, F-75015 Paris (FR)**

(74) Mandataire: **Gutmann, Ernest, S.C. Ernest Gutmann - Yves Plasseraud 67, boulevard Haussmann, F-75008 Paris (FR)**

## Description

L'invention concerne un procédé de séparation ou de purification d'un facteur favorisant le sommeil à partir d'un milieu biologique, ou d'enrichissement de ce milieu en un tel facteur.

La présence d'un facteur favorisant le sommeil, dénommé facteur "S", dans des milieux biologiques tels que l'urine humaine, est connue. Un procédé de séparation de ce facteur S à partir de l'urine humaine a été décrit par J. M. KRUEGER et coll. dans un article publié sous le titre "Compositions d'un facteur favorisant le sommeil, isolé à partir d'urine humaine" (J. Biol. Chem., 1982, vol. 257, 1 665-1 669). Cet article indique également que ce glycopeptide paraissait avoir une analogie de structure avec des peptidoglycane bactériens, notament en raison de l'analogie de certains de leurs constituants et des rapports molaires existant entre eux. L'analyse du facteur S purifié a en effet révélée qu'il contenait de l'acide glutamique, de l'alanine, de l'acide diaminopimélique et de l'acide muramique, dans des rapports molaires de 2 : 2 : 1 : 1.

L'article rapporte également des résultats d'essais biologiques ayant mis en jeu l'administration intraventriculaire de quantités picomolaires du facteur S chez l'animal. On a observé l'induction par ce facteur chez le rat, le lapin et le chat ainsi traités, d'un sommeil prolongé marqué par une production d'ondes lentes repérables par électro-encéphalographie. Le someil ainsi induit présente toutes les caractéristiques du sommeil réparateur normal, quelquefois désigné sous l'expression anglaise"Slow Wave Sleep" (SWS: someil à ondes lentes).

Le procédé décrit dans cet article qui paraît représenter à ce jour la technique connue, la plus efficace pour isoler ce facteur S, implique cependant un nombre d'étapes considérable (10 étapes successives), procédé d'autant plus difficile à mettre en oeuvre qu'il implique le traitement de volumes considérables du milieu biologique. En effet, pour obtenir 30 µg de facteur S, il a fallu traiter un volume initial de 5000 l d'uriné humaine.

L'invention a pour but de fournir un procédé à la fois simplifié et sélectif pour obtenir ce facteur S, ou tout facteur en présentant les caractéristiques, à partir de tout milieu biologique le contenant.

L'invention decoule de la découverte que ce facteur pouvait être reconnu de façon sélective par des anticorps anti-MDP (abréviation de N-acétyl-muramyl-L-alanyl-D-isoglutamine) spécifiques.

Le procédé selon l'invention de séparation ou de purification du facteur S, à partir d'un milieu biologique liquide le contenant à l'état dissous, ou d'enrichissement de ce milieu en ce facteur S, ledit milieu biologique liquide étant d'origine humaine ou animale, notamment de l'urine humaine ou du liquide céphalorachidien de chèvre, est caractérisé par la mise en contact de ce milieu avec des anticorps reconnaissant le groupe N-acétyl-muramique et le premier résidu aminoacyle contenu dans la chaîne peptidique de la N-acétyl-muramyl-L-alanyl- Disoglutamine ou des dérivés de l'acide N-acétyl-muramyl-L-alanyl- D-glutamique ou encore d'homologues des précédents composés dans lesquels le groupe L-alanyle est remplacé par un groupe L-valyle ou L-séryle, et par la récupération du facteur S lui-même à partir du complexe facteur S-anticorps.

Les anti-corps anti-MDP susceptibles d'être mis en oeuvre dans le prodédé de purification selon l'invention peuvent être obtenus de toute façon en soi connue, notamment par immunisation de l'animal, par exemple le lapin, avec un MDP ou dérivé de MDP préalablement fixé sur une molécule porteuse, telle que la sérumalbumine bovine, des polylysines ou autre molécule ayant un poids moléculaire suffisamment élevé pour conférer au conjugué obtenu l'immunogénicité requise. En ce qui concerne les conditions générales de la fabrication des anticorps anti-MDP, on peut se référer à l'article de REICHERT C. M. et coll. publié dans Molec. Immun. 17, 357-363 (1980). On peut aussi se reporter à l'article de BAHR J. M. et coll. publié dans Molec. Immun. 19, n° 5, 737-745 (1982).

Les anticorps contenus dans les antisérums obtenus peuvent être purifiés, par exemple par chromatographie affine sur une colonne de résine portant des molécules de MDP ou dérivées de MDP fixées sur cette résine. Avantageusement, on aura recours à une colonne du matériau support insoluble commercialisé sous la désignation SEPHAROSE 4B® sur laquelle ont été fixés des groupes MDP-lysine (N-acétyl-muramyl-L-alanyl-D-isoglutaminyl-L-lysine), après activation préalable du matériau support au bromure de cyanogène.

Les anticorps anti-MDP (anticorps anti-MDP polyclonaux) fixés sur un tel matériau peuvent ensuite être élués par exemple par acidification du milieu, notament à un pH inférieur à 4, ou par accroissement de la concentration ionique du milieu, grâce à des sels ioniques fortement solubles dans l'eau, tels que le thiocyanalie de sodium.

Selon une caractéristique préférée importante de l'invention, on a cependant recours à des anticorps anti-MDP "sélectifs" en ce qu'ils reconnaissent de façon spécifique la structure N-acétyl-muramyl-L-alanyl-D-isoglutamine dans son ensemble, à l'exclusion notamment soit de la structure de l'acide N-acétyl-muramique isolé, soit de celle de la L-alanyl-D-isoglutamine isolée.

Ces derniers anticorps anti-MDP sélectifs, plus particulièrement des anticorps monoclonaux, permettront un degré de purification nettement plus poussé que les anticorps polyclonaux, puisque ces derniers sont susceptibles de fixer d'autres constituants des milieux biologiques concernés, par exemple de nombreuses autres structures glycopeptides également présentes dans ces milieux. Néanmoins les anticorps anti-MDP polyclonaux peuvent être utilisés avec avantage pour produire un premier enrichissement en facteur S, des purifications plus poussées étant ensuite, si besoin, répétées en mettant en oeuvre des anticorps "sélectifs" monoclonaux. Une technique d'obtention- d'hybridomes secrétant de tels anticorps monoclonaux et de récupération de ceux-ci à partir des milieux de cultures de ces hybridomes, ainsi que les conditions dans lesquelles ces anticorps monoclonaux peuvent être mis en oeuvre, seront indiquées plus loin, exclusivement à titre d'exemples.

Le procédé selon l'invention présente donc les caractéristiques essentielles que constitue la formation de complexes entre le facteur S et les anticorps anti-MDP, de préférence les anticorps "sélectifs", et la récupération du facteur S lui-même à partir de ce complexe, après dissociation de celui-ci.

Il est avantageux d'avoir recours à des anticorps sélectifs anti-MDP supportés par un support insoluble en milieu aqueux. A cet effet, on peut avoir recours à tout support, de préférence pulvérulent, sur lequel les anticorps anti-MDP peuvent être fixés sans que soit mise en cause leur capacité de former des complexes avec le facteur S isolé. On préfére avoir recours à des anticorps anti-MDP sélectifs fixés sur le matériau support commercialisé sous la désignation SEPHAROSE 4B®, après activation de celui-ci au bromure de cyanogène. Bien entendu, d'autres supports peuvent être envisagés tels que billes de verre, agarose, polyacrylamides et SEPHADEX® étant naturellement entendu qu'il appartiendra au spécialiste de procéder aux essais de fixation des anticorps sélectifs anti-MDP et de vérification de la préservation de l'activité complexante des anticorps anti-MDP ainsi fixes.

Les opérations de récupération du facteur S à partir du complexe préalablement formé entre celui-ci et des anticorps sélectifs anti-MDP peuvent être conduites de toute façon en soi connue, par exemple par acidification du milieu, notament à un pH inférieur à 4, ou par accroissement de la concentration ionique du milieu grâce à des sels ioniques fortement solubles par exemple tels que le thiocyanate de sodium le chlorure de sodium ou le thiocyanate d'amonium. Ces méthodes sont particulièrement avantageuses, surtout lorsque les anticorps anti-MDP auront au préalable été fixés sur un support insoluble approprié. On peut avantageusement utiliser un anticorps anti-MDP obtenu à pàrtir d'une solution d'anticorps anti-MDP polyclonaux, préalablement purifiée par chromatographie affine sur une colonne de SEPHAROSE 4B-MDP-lysine, les anticorps anti-MDP étant ensuite élués de la colonne, notamment dans les conditions qui ont été indiquées plus haut. D'une façon générale, on peut avoir recours, pour la fixation des anticorps anti-MDP sur support insoluble, à toute technique classique de fixation d'anticorps de ce type, par exemple telle que décrite dans "Chromatographie d'affinité: Principes et Méthodes", Pharmacia.

De préférence, le milieu biologique initial dont le facteur S peut être extrait est constitué par de l'urine humaine. Bien entendu, on n'exclut pas d'autres milieux possibles d'origine humaine ou animale, par exemple le liquide céphalorachidien ou CSF (abréviation anglaise de "cerebrospinal fluid") originaire de la chèvre.

Il est naturellement avantageux de concentrer autant que possible le milieu biologique traité, avant sa mise en contact avec les anticorps anti-MDP, notament pour le débarrasser des protéines ayant des poids moléculaires élevés, par exemple supérieurs à une valeur de l'ordre de 25.000, voire supérieure à 1.000-2.000. De preférence encore, le milieu biologique traité contenant le facteur S peut être débarrassé au préalable des glycopeptides, peptides ou protéines, ayant des caractéristiques biochimiques distinctes de celles du facteur S, par exemple des protéines non fixables sur résines échangeuses d'anions, par exemple agglutinables par des anticorps ne donnant aucune réaction croisée avec le facteur S. Il peut être avantageux d'avoir recours aux milieux concentrés et enrichis, obtenus à partir de l'urine humaine, au terme des quatre premières étapes successives du procédé décrit par J.M. KRUEGER et Coll.. Une méthodologie utilisable pour réaliser la séparation ou la purfication du facteur S sera exposée plus loin.

L'invention sera davantage encore illustrée par la description des techniques qui ont permis la détermination de la capacité de reconnaissance du facteur S ou de facteurs analogues par des anticorps sélectifs anti-MDP.

## 1° Production d'anticorps monoclonaux

### a) Immunisation des souris

Deux groupes de souris femelles BALB/c, âgées de deux mois, ont été imunisés avec du MDP préalablement fixé sur de la sérumalbumine bovine (MDP-BSA). Le premier groupe reçoit deux injections intradermiques décalées l'une de l'autre par un intervalle de trois semaires, de 100 µg de MDP-BSA émulsifiés avec de l'adjuvant complet de FREUND (CFA). Le second groupe subit le même traitement, par voie intrapéritonéale, et avec des doses de 100µg de MDP-BSA dans le tampon PBS.

### b) Fusion des cellules et culture des hybrides cellulaires (hybridomes)

Neuf semaines après la dernière imunisation, les souris reçoivent deux rappels à un jour d'intervalle, avec 100 µg de MDP-BSA, en milieu salin, cette fois-ci par voie intrapéritonéale. Trois jours après le dernier rappel, on sacrifie les souris, récupère les cellules spléniques et réalise une fusion cellulaire de ces cellules spléniques avec une lignée de cellules de myélome, par exemple du myélome NSO. Il va naturellement de soi que l'on peut utiliser tout autre type accessible de cellules de myélome susceptibles de former, par fusion avec des cellules spléniques, des hybrides cellulaires capables d'induire la production d'ascites chez l'animal alors susceptibles d' être utilisés come source des anticorps présentant les caractéristiques de ceux initialement produits par les cellules spléniques mises en jeu dans la fusion.

La fusion cellulaire de 100 millions de cellules spléniques de chaque groupe de souris avec $10 \times 10^6$ cellules NSO/1 en présence d'une solution de polyéthylène glycol 1 500 à 41 %, peut être réalisée selon la technique décrite par Z. ESHHAR et coll. dans J. Immunol. 124, 775, 1980. Après fusion, les cellules sont distribuées dans trois à quatre microplaques à 96 puits, puis sélectionnées dans un milieu de EAGLES, modifié par DULBECCO, contenant les constituants caractéristiques du milieu HAT, ayant une teneur en hypoxanthine, aminoptérine et thymidine (HAT-DMEM) et une forte teneur en glucose, milieu qui en outre est complété pour contenir 15 % de sérum de cheval, 50 unités par ml de pénicilline et de streptomycine, 1 mM de pyruvate de sodium et 2 mM de glutamine.

Après deux semaines de culture, les hybrides cellulaires sont transférés dans un milieu HT-DMEM. Les cellules qui se sont développées, peuvent ensuite être cultivées en routine dans un milieu à base seulement de DMEM et de sérum de cheval.

**Tri des clônes fabriquant des anticorps anti-MDP sélectifs**

De 10 à 15 jours après l'opération de fusion, on fait le tri des hybrides susceptibles de produire des anticorps anti-MDP, en ayant recours à la technique de "radioimunoassay" en phase solide, sur microplaques de poly(chlorure de vinyle) dont les puits avaient au préalable été revêtus avec du MDP fixé sur multi(poly-D-L-alanyl)(poly-L-lysine): MDP-A-L, à raison de 100 µl d'une solution de 25 µg/ml de MDP-AL par puits. Après incubation à la température ambiante, pendant une heure, les microplaques sont lavées trois fois avec une solution de sérum de cheval à 1 % dans le tampon PBS (PBS-HS). 50 µl de surnageants des cultures d'hybridomes sont alors ajoutés dans chacun des puits et incubés pendant 2 heures à la température ambiante. Après trois lavages avec PBS-HS, on ajoute 50 µl d'anticorps Ig de chèvre anti-souris, marqués à l'iode 125 ($^{125}$I) ($10^5$ coups par minute: cpm). On laisse incuber pendant une nuit à 4°C. Le niveau de radio-activité est déterminé dans un compteur de rayonnements Gamma après 4 lavages, séchages et découpages des puits.

Le tableau qui suit résume les "efficacités de fusion" des deux essais de fusion mentionnés plus haut.

| Groupe | Immunisation | Nombre de cultures de cellules hybrides qui ont poussé | Hybridomes positifs |
|--------|--------------|--------------------------------------------------------|---------------------|
| 1 | MDP-BSA dans le CFA | 384/384 | 72/384 |
| 2 | MDP-BSA dans le PBS | 288/288 | 29/288 |

Ont été considérés comme hybridomes positifs ceux qui ont conduit à des détections de 5 000 à 15 000 cpm, eu égard à un bruit de fond de 500 cpm dans les essais réalisés avec des surnageants non producteurs d'anticorps anti-MDP.

On a réalisé une deuxième sélection d'anticorps monoclonaux anti-MDP dans des conditions semblables dans une deuxième série d'essais avec des microplaques dont les puits avaient été revêtus respectivement de MDP-A-L, de M-A-L et DP-A-L (c'est-à-dire de composés dans lesquels le MDP est remplacé par ses constituants de base: acide muramique (M) et L-alanyl-D-isoglutamine (DP)). Les anticorps monoclonaux actifs à l'égard de MDP-A-L, qui ne présentaient cependant que peu ou pas d'activité à l'égard de DP-A-L ou de M-A-L, ont été sélectionnés.

Pour produire des quantités d'anticorps plus importantes, les hybridomes sélectionnés sont administrés par voie intrapéritonéale à raison de $10^7$ cellules hybrides à des souris BALB/c x DBA/2)F1. Les anticorps secrétés sont ensuite récupérés de façon connue en soi à partir des tumeurs ascitiques fluides induites par ces hybridomes chez ces souris, notamment en fractionnant l'ascite sur un gel, en particulier le gel SEPHACRYL 200® et en récupérant la bande contenant l'imunoglobuline qui donne lieu à des rèactions immunologiques croisées avec l'antigène MDP (bande correspondant à un poids moléculaire d'environ 160.000).

**Mise en évidence de la capacité des anticorps anti-MDP de se combiner au facteur S**

On a recours à la technique antérierurement décrite dans l'article de BAHR et Coll. déjà mentionné, sous réserve de quelques modifications de détail. Les éléments essentiels de la méthodologie utilisée sont rappelés

**0 100 734**

ci-après.

Les puits de plaques micro-ELISA® (commercialisées par la société DYNATECH) sont tapissés avec des fractions Ig d'anticorps de chèvre anti-souris IgM, IgA ou IgG, par maintien en contact avec leurs parois d'une solution contenant 5 µg/ml de la fraction Ig pendant 3 heures à 4°C. Après lavage de la plaque avec une solution de PBS contenant 0,05 % de l'agent de dispersion commercialisé sous la dénomination TWEEN 20, on ajoute des dilutions des surnageants d'hybridomes producteurs d'anticorps anti-MDP aux différents puits et on incube pendant 2 heures à la température ambiante. Les fractions Ig facilitent la retenue des anticorps anti-MDP. Les plaques sont ensuite lavées et une solution de MDP-lysine couplé à de la péroxydase de raifort diluée au 1/1000 est ajoutée aux puits. Les plaques sont ensuite laissées au repos pendant la nuit à 4°C. Après lavage, on détermine les proportions de MDP-lysine-péroxydase fixées dans les puits, en mettant en oeuvre l'ortho-phénylène-diamine et de l'eau oxygénée en tant que substrat de la péroxydase. La réaction est ensuite arrêtée par addition d'une solution de $H_2SO_4$ à 12,5% et la densité optique est lue à 492 nm dans la photomètre TITERTEK MULTISKAN ELISA (FLOW).

Dans ce qui suit, la valeur de densité optique, qui est obtenue à l'occasion de la fixation réalisée après mise en contact de la MDP-péroxydase diluée au 1/1000 avec l'anticorps monoclonal anti-MDP dilué au 1/1000, sera considérée comme correspondant à une activité de 100%.

La capacité du facteur S à être complexé par les anticorps monoclonaux anti-MDP est appréciée par son aptitude à inhiber la fixation du conjugué MDP-péroxydase, lorsqu'il est ajouté dans les puits desdites microplaques en même temps que le conjugué MDP-péroxydase dans les conditions qui ont été indiquées plus haut.

Ainsi a-t-on obtenu des inhibitions exprimées par des activités:
- de 31% lorsque 100 µ1 d'une solution de MDP contenant 100 ng du facteur S a été ajoutée an même temps que le -conjugué;
- de 24 % lorsque 100 µ1 d'un extrait de cerveau contenant 140 ng du facteur S a été ajouté en même temps que le conjugué,
- de 18 et 15% respectivement lorsque, en même temps que le conjugué, ont été ajoutés respectivement 70 ng et 10 ng du facteur S extrait d'un échantillon d'urine humaine, dans les conditions qui ont été évoquées plus haut.

L'addition dans les mêmes conditions de N-acétylmuramyl-D-alanyl-D-isoglutamine (MDP-DD) ne produit aucune inhibition. De l'ensemble des résultats qui ont été indiqués, il apparait que le facteur S témoigne d'une grande efficacité de fixation sur les anticorps monoclonaux sélectifs anti-MDP.

Il découle de ce qui précède que cette réaction de complexation sélective du facteur S peut être mise à profit pour le séparer à partir d'un milieu le contenant, en mélange avec d'autres peptides, glycopeptides et en particulier fragments de peptidoglycanes. Il est à cet égard remarquable de noter que les anticorps anti-MDP ne conduisent à aucuné réaction immunologique avec les peptidoglycanes, à moins qu'ils ne contiennent la structure MDP convenablement exposée.

Le facteur S, tel qu'il est retenu dans les complexes facteur S - anticorps anti-MDP, peut être obtenu à partir de celui-ci par dissociation dudit complexe. Mais comme on l'a indiqué plus haut, cette dissociation pourra être réalisée beaucoup plus simplement si les anticorps anti-MDP sont utilisés sous une forme supportée sur un support insoluble.

On décrit ci-après une méthodologie susceptible d'être utilisée pour procéder à une séparation de quantités plus importantes de facteur S à partir d'urine humaine:

a) On utilise une colonne de SEPHAROSE 4B activée au bromure de cyanogène couplé selon des techniques classiques à des anticorps monoclonaux anti-MDP.

b) Purification de lots d'urine.

Partant de lots d'urine, contenant des agents de conservation classiques, on leur fait subir les quatre étapes A, B, C et D visées dans l'article de J.N. KRUEGER et al, lequel est considéré comme faisant partie de la présente description, dans la mesure où des éléments de l'état de la technique sont nécessaires à la description complète de l'invention. Ces étapes consistent en: A - une extraction de l'urine sur carboxyméthyle-SEPHADEX G-25®,

B - une gel-filtration sur SEPHADEX-G-10®,

C - une chromatographie d'échanges d'anions sur DEAE-SEPHADEX® et

D - une gel-filtration de nouveau sur SEPHADEX-G-10®.

Bien entendu, on peut avoir recours à toutes autres techniques de séparation des protéines à poids moléculaires élevés, telles que filtration et dessalage sur colonne de SEPHADEX-G-25.

c) Immuno-absorption du facteur S.

L'urine partiellement purifiée et concentrée, obtenue au termes des étapes précédentes, est mise en contact avec une colonne immuno-absorbante retenant des groupes anti-MDP fixés. Après un contact suffisant à permettre la fixation sélective du facteur S, le matériau imuno-absorbant est lavé pour éliminer les produits retenus non spécifiques. Avantageusement, on travaille sur colonne, et les rinçages sont effectués par le passage du liquide de rinçage au travers de la colonne. Les molécules retenues sur les anticorps de la colonne, c'est-à-dire le facteur S et éventuellement d'autres monokines ou médiateurs susceptibles de contenir la structure MDP sous une forme accessible aux anticorps, sont élués par un tampon à bas pH, par exemple une solution à base de glycine/HCl, pH 3,2 ou par une solution à concentration saline élevée, par exemple le thiocyanate de sodium 1M.

d) Pour le cas où d'autres molécules seraient éluées en même temps que le facteur S, il sera souhaitable de séparer ces molécules par chromatographie sur tamis moléculaire permettant des séparations de molécules selon leurs poids moléculaires.

Parmi les molécules éventuellement présentes, on mentionne des facteurs pyrogènes, endogènes, lesquels auront un poids moléculaire généralement plus élevé. En conséquence, il peut être souhaitable d'avoir recours à une séparation, notament des molécules ayant un poids moléculaire supérieur à 5000, et celles ayant un poids moléculàire inférieur à ces valeurs.

Cette séparation peut également être réalisée par contact des éluants avec d'autres matériaux immunoabsorbants, notamment en colonne, comportant des anticorps spécifiquement dirigés contre ces autres molécules, plus particulièrement des anticorps anti-pyrogènes endogènes.

Ces procédures sont particulièrement favorables, puisque les élutions permettent en même temps la régénération des supports imuno-absorbants. Une colonne de 3 ml de SEPHAROSE 4B anti-MDP devrait permettre, par le jeu des fixations et régénérations alternées successives, la séparation de plusieurs mg de facteur S.

D'une façon générale, les anticorps monoclonaux susceptibles d'être mis en oeuvre dans le procédé selon l'invention peuvent être constitués par tout anticorps monoclonal reconnaissant sélectivement les homologues du MDP, caractérisé par la présence d'une chaîne peptidique liée au groupe acide N-acétyl-muramique, le premier aminoacyle de la chaîne peptidique, c'est-à-dire l'aminoacyle assurant la liaison avec le groupe N-acétyl-muramique, étant choisi parmi L-alanyle, L-séryle ou L-valyle. Le deuxième aminoacyle de la chaîne peptidique n'intervient guère dans la spécificité du déterminant antigénique important à prendre en considération, pour ce qui est des anticorps monoclonaux susceptibles d'être mis en oeuvre.

Les anticorps monoclonaux particulièrement sélectifs sont ceux qui, parmi ceux qui viennent d'être définis, ne reconnaissent pas la structure acide N-acétyle muramique dépourvue du groupement peptidique susindiqué, même à des concentrations 100 fois plus importantes que la concentration minimale de l'anticorps monoclonal correspondant nécessaire à la formation du complexe dérivé de MDP répondant à la condition sus-indiquée.

Il résulte donc bien de ce qui précède que les anticorps monoclonaux susceptibles d'être mis en oeuvre peuvent également être obtenus à partir d'hybridomes dont la production met initialement en oeuvre des fusions de cellules de myélomes avec des splénocytes d'animaux immunisés avec les susdits dérivés ou homologues de MDP dans lesquels les fonctions $\alpha$ et $\gamma$ -carboxyle peuvent porter des substituants différents, en particulier avec des diesters de l'acide N-acétylmuramyl-L-alanyl-D-glutamique ou encore les $\alpha$ -esters de la N-acétylmuramylL-alanyl-D-glutamine, tels qu'ils ont été décrits dans la demande de brevet européen n° 79 400 357.3

En ce qui concerne enfin la fabrication des hybridomes, il va de soi que l'on peut avoir recours à tout type de myélome couramment utilisé à cet effet. En particulier, on aura recours à tout myélome décrit dans la littérature technique ou dans des demandes de brevets ou brevets publiés. On mentionnera, à titre d'exemple, les myélomes plus particulièrement décrits dans les demandes de brevets européens n° 0 014 519, N° 0 018 794, N° 0 018 795 et la demande de brevet français publiée N° 78 17545/2.394.607.

## Revendications

1. Procédé de séparation ou de purification du facteur S, à partir d'un milieu biologique liquide le contenant à l'état dissous, ou d'enrichissement de ce milieu en ce facteur S, ledit milieu biologique liquide étant d'origine humaine ou animale, notamment de l'urine humaine ou du liquide céphalorachidien de chèvre, ce procédé étant caractérisé par la mise en contact de ce milieu avec des anticorps recònnaissant le groupe N- acétyl-muramique et le premier résidu aminoacyle contenu dans la chaîne peptidique de la N-acétyl-muramyl- L-alanyl-D-isoglutamine ou des dérivés de l'acide N-acétyl-muramyl-L-alanyl- D-glutamique, ou encore d'homologues des précédents composés dans lesquels le groupe L-alanyle est remplacé par un groupe L-valyle ou L-séryle, et par la récupération du facteur S lui-même à partir du complexe facteur S-anticorps.

2. Procédé selon la revendication 1, caractérisé en ce que les anticorps mis en oeuvre sont essentiellement constitués par des anticorps reconnaissant spécifiquement la structure N-acétylmuramyl-L-alanyl-D-isoglutamine dans son ensemble, mais pas la structure isolée de l'acide N-acétylmuramique isolée ou de celle isolée de la L-alanyl- D- isoglutamine.

3. Procédé selon la revendication 2, caractérisé en ce que les anticorps sont des anticorps monoclonaux.

4. Procédé selon l'une quelconque des revendications 1 à 3, caractérisé en ce que les anticorps sont fixés sur un support insoluble en milieu aqueux.

5. Procédé selon l'une quelconque des revendications 1 à 4, caractérisé en ce que l'opération de récupération du facteur S, à partir du complexe facteur S-anticorps, comprend la mise en contact de ce complexe avec un milieu tamponné acidifié à pH inférieur à 4, par accroissement de la concentration ionique du milieu.

6. Procédé selon la revendication 5 caractérisé en ce que l'accroissement de concentration ionique du milieu est réalisée par l'addition au milieu de sels ioniques, fortement solubles tels que le thiocyanate de sodium, le chlorure de sodium ou le thiocyanate d'ammonium.

7. Procédé selon l'une quelconque des revendications 1 à 6, caractérisé en ce que le milieu biologique contenant le facteur S initialement mis en oeuvre, est constitué par de l'urine humaine ou par un concentré d'urine humaine.

6. Procédé selon la revendication 7, caractérisé en ce que le concentré d'urine humaine est dépourvu des protéines ayant un poids moléculaire supérieur à une valeur de l'ordre de 25 000.

9. Procédé selon la revendication 7, ou la revendication 6, caractérisé en ce que le concentré d'urine humaine a été débarrassé au préalable des protéines non fixables sur résines échangeuses d'anions.

## Patentansprüche

1. Verfahren zur Abtrennung oder Reinigung des Faktors S aus einem flüssigen, biologischen Medium, in dem er in gelöstem Zustand enthalten ist, oder zur Anreicherung des Faktors S aus diesem Medium, wobei das flüssige, biologische Medium vom Menschen oder Tier stammt, vorzugsweise aus menschlichen Urin oder der Gehirn-Rückenmarks-Flüssigkeit der Ziege, dadurch gekennzeichnet, daß man dieses Medium in Kontakt bringt mit Antikörpern gegen die N-Acetylmuramin-Gruppe und den ersten Aminoacylrest in den peptiden N-Acetylmuramyl-L-alanyl-D-isoglutamin oder Derivate der N-Acetyl-muramyl-L-alanyl-D-glutaminsäure oder Homologe dervorgenannten Verbindungen, in denen die L-Alanylgruppe durch eine L-Valyl oder L-Seryl-Gruppe ersetzt ist, und den Faktor S aus dem Faktor S-Antikörper-Komplex gewinnt.

2. Verfahren nach Anspruch 1, dadurch gekennzeichnet, daß die eingesetzten Antikörper im wesentlichen aus spezifischen Antikörpern gegen die Anordnung N-Acetylmuramyl-L-alanyl-D-isoglutamin als Ganzes, aber nicht gegen die Einzelstrukturen N-Acetylmuraminsäure oder L-Alanyl-Disoglutamin bestehen.

3. Verfahren nach Anspruch 2, dadurch gekennzeichnet, daß die Antikörper monoclonale Antikörper sind.

4. Verfahren nach einen der Ansprüche 1 - 3, dadurch gekennzeichnet, daß die Antikörper an einen in einem wäßrigen Medium unlöslichen Träger gebunden sind.

5. Verfahren nach einen der Ansprüche 1 - 4, dadurch gekennzeichnet, daß man zur Gewinnung des Faktors S aus den Faktor S-Antikörper-Komplex den Komplex in ein durch Erhöhen der Ionenkonzentration unter einen pH-Wert von 4 angesäuertes Medium einbringt.

6. Verfahren nach Anspruch 5, dadurch gekennzeichnet, daß die Erhöhung der Ionenkonzentration des Mediums durch Zusatz von gutlöslichen Salzionen, wie Natriummthiocyanat, Natriumchlorid oder Ammoniumthiocyanat erfolgt.

7. Verfahren nach einem der Ansprüche 1 - 6, dadurch gekennzeichnet, daß das den Faktor S enthaltende biologische Medium, das als Ausgangsmaterial verwendet wird, menschlicher Urin oder konzentrierter menschlicher Urin ist.

8. Verfahren nach Anspruch 7, dadurch gekennzeichnet, daß der konzentrierte menschliche Urin keine Proteine mit einem Molekulargewicht über 25000 enthält.

9. Verfahren nach Anspruch 7 oder 8, dadurch gekennzeichnet, daß aus dem konzentrierten menschlichen Urin vorher die Proteine entfernt wurden, die nicht an Anionenaustauscherharze binden.

## Claims

1. Process of separation or of purification of factor S, from a liquid biological medium containing it in the dissolved state, or of factor S enrichment of this medium, the said liquid biological medium being of human or animal origin, in particular human urine or cephalorachidian fluid from goats, this process being characterized by the fact of bringing this medium into contact with antibodies recognizing the N-acetyl-muramic group and the first aminoacyl residue contained in the peptidic chain of N-acetyl-muramyl- L-alanyl D-isoglutamine or derivatives of N-acetyl-muramyl L-alanyl D-glutamic acid or homologues of the said compounds in wnich the L-alanyl group is replaced by an L-valyl or L-seryl group, anb by the recovery of the factor S itself from the factor S-antibodies combination.

2. Process according to claim 1, characterized by the fact that the antibodies involved consist essentially of antibodies specifically recognizing the N-acetyl-muramyl L-alanyl D-isoglutamine structure as a whole, but not the structure of N-acetylmuramic acid in isolation or the isolated structure of L-alanyl-D-isoglutamine.

3. Process according to claim 2, characterized by the fact that the antibodies are monoclonal antibodies.

4. Process according to any of claims i to 3, characterized by the fact that the antibodies are fixed to a support insoluble in an aqueous medium.

5. Process according to any of claims 1 to 4, characterized by the fact that the operation of recovering the factor S from the factor S-antibodies combination consists of bringing this combination into contact with a buffer medium acidified to a pH of less than 4, hy increasing the ionic concentration of the medium.

6. Process according to claim 5, characterized by the fact that. the increase in ionic concentration of the medium is achieved by adding to the medium highly soluble ionic salts such as sodium thiocyanate, sodium chloride or ammonium thiocyanate.

7. Process according to any of claims 1 to 6, characterized hy the fact that the biological medium containing the factor S initially used consists of human urine or of a human urine concentrate.

8. Process according to claim 7, characterized by the fact that the human urine concentrate is free from proteins having a molecular weight higher than a value of the order of 23 000.

9. Process according to claim 7 or claim 8, charactcrized by the fact that the human urine concentrate has previously had removed from it proteins which are non-fixable on anion exchange resins.